# EUROPÄISCHE PATENTANMELDUNG

(11) **EP 2 468 237 A2**
(43) Veröffentlichungstag der Anmeldung: **27.06.2012**
(21) Anmeldenummer: 11182081.7
(22) Anmeldetag: 21.09.2011
(51) Int. Cl.: A61K 8/04, A61K 8/34, A61K 8/41, A61K 8/49, A61K 8/891, A61K 8/898, A61Q 5/12

(54) **Haarnachbehandlungsmittel in Sprayform (leave-in) mit Imidazolinen**

(30) Priorität: 28.09.2010 DE 102010041496
(71) Anmelder: Beiersdorf AG, 20253 Hamburg (DE)
(72) Erfinder: Saladin, Sandra, 20144 Hamburg (DE); Ölrichs, Ilka, 25436 Tornesch (DE)

(57) **Zusammenfassung**

Haarnachbehandlungsmittel in Form einer sprühbaren Hydrodispersion mit Viskosität kleiner als 1000mPas [gemessen mit dem Rheomat R 123 proRheo, Messkörper 1 (Art.Nr. 200191)] enthaltend kationische Imidazoline der Formel 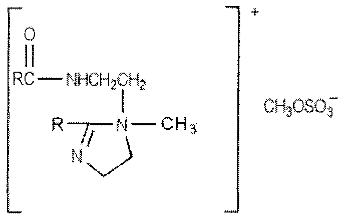 bevorzugt diejenigen, bei denen R ein aliphatischer Rest aus dem Triglycerid Palmöl ist, in Kombination mit Monoalkyltrimethylammonium Salzen, langkettigen Fettalkoholen einem erstem Silikonöl und einem weiteren Silikonöl, das getrennt von dem ersten Silikonöl zu der Hydrodispersion hinzugefügt wird.

## Beschreibung

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie auch die Verwendung von Haarpflegemitteln, die nicht ausgespült werden. Diese Haarnachbehandlungsmittel kommen in verschiedenen Technologieformen vor, wie z.B. dünne O/W Emulsionen, Hydrodispersionen, wässrige-alkoholische Zubereitungen.

Quaternäre Ammoniumverbindungen vom Typ der Mono-, Di- und/oder Trialkylammoniumverbindungen sind seit langem bekannt. Besonders C₂₂-MonoalkylTrimethylammonium Verbindungen unter Incroquat Behenyl TMC-Typen von Croda und Genamin KDMP von Clariant führen zu der vom Verbraucher gewünschten Pflegeleistung wie z. B. Geschmeidigkeit der Haare, gute Nass- und Trockenkämmbarkeit, und verbesserte Haarstruktur, jedoch nicht ohne den Nachteil von fettigen Haaren je nach Einsatzkonzentration.

Bekannt ist, dass Imidazolinderivate mit mindestens zwei langen Fettketten zu einer verbesserten Pflege von Haaren führen. Diese haben den weiteren Vorteil der biologischen Abbaubarkeit. Durch deren Einsatz wird die vom Verbraucher erwünschte Pflegeleistung jedoch nicht erreicht.

Die Schrift DE-102008031749 offenbart eine kosmetische Zubereitung mit mindestens einem Imidazolinderivat und einem Esteröl. Mehrere Imidazolinquats werden in einer Formel zusammengesetzt, um die erwünschte Pflegeleistung zu erzielen.

Die Schrift DE-102008031748 offenbart ein haarkonditionierendes Mittel enthaltend mindestens 0.01 Gew% eines quaternären Imidazolinderivates mit einer kationisch geladenen polymeren Verbindung und einem kosmetischen Träger.

Die Schrift DE-102007060528 A1 offenbart kosmetische Zubereitungen mit Imidazolinderivaten und Silikonölen in einem bestimmten Verhältnis.

Die Schrift DE-102008031702 A1 offenbart kosmetische Zubereitungen, die mindestens ein Imidazolinderivat und ein Monoalkyltrimethylammonium Salz enthalten.

Überraschenderweise wurde gefunden, dass ein Haarnachbehandlungsmittel in Form einer sprühbaren Hydrodispersion mit Viskosität kleiner als 1000 mPas [gemessen mit dem Rheomat R 123 proRheo, Meßkörper 1 (Art.Nr. 200191)] enthaltend kationische Imidazoline (auch Imidazolinquat genannt) der Formel 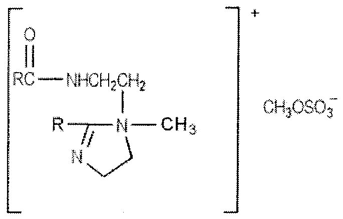 bevorzugt diejenigen, bei denen R ein aliphatischer Rest aus Palmöl ist, in Kombination mit Monoalkyltrimethylammonium Salzen, langkettigen Fettalkoholen einem erstem Silikonöl und einem weiteren Silikonöl, das getrennt von dem ersten Silikonöl zu der Hydrodispersion hinzugefügt wird, den Nachteilen des Stand der Technik abhilft. Ein solches Mittel führt zu einer überragenden Pflegeleistung. Beim Einsatz dieser Kombination kommt es zu überraschend guten Eigenschaften der behandelten Haare, insbesondere zu verbesserten Kämmbarkeiten, zu verbessertem Glanz, Geschmeidigkeit der Haare ohne zu beschweren. Das Produkt wird aufs handtuchtrockene Haar aufgesprüht und nicht mehr ausgespült. Besondere Vorteile bietet diese Erfindung im Gegensatz zum Stand der Technik signifikant überlegene Pflege für langes, beanspruchtes europäisches Haar. In einem monadischen Verbrauchertest an schulter-langen und längeren trocken/strapazierten Haaren in Deutschland zeigte die hier genannte Formulierung klare Vorteile hinsichtlich konsumentenrelevante Parameter: Das Produkt erwiese sich als besonders mild zu Haar und Kopfhaut, schützte das Haar besonders vor dem Austrocknen, trocknete das Haar in besonderer Weise nicht aus, verlieh dem Haar ein besonders gesundes Erscheinungsbild, bewahrte in besonderer Weise die Gesundheit von Kopfhaut und Haar, verlieh dem Haar einen besonderen seidigen Glanz, machte das Haar besonders weich und geschmeidig, verbesserte das Haargefühl nach dem Trocknen, pflegte das Haar besonders intensiv, erwies sich als besonders geeignet für langes, strapaziertes Haar, erweichte die Haaroberfläche in besonderer Weise, war besonders wirksam gegen Spliss, war besonders geeignet für geschädigtes Haar, schützte besonders gegen elektrostatische Aufladung und Kräuseln, beugte besonders der Schädigung des Haares vor, verbesserte besonders die Naßanfühleigenschaften des behandelten Haares, verbesserte besonders die Nasskämmbarkeit des Haares, verbesserte besonders die Trockenkämmbarkeit des Haares, verbesserte in besonderer Weise die Frisierbarkeit des Haares, hinterließ besonders wenig Rückstände auf dem Haar, verlängerte in besonderer Weise die natürliche Haarelastizität.

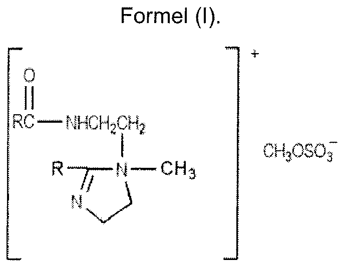

Ein bevorzugtes Handelsprodukt für Formel (I) ist beispielsweise unter der INCI-Bezeichnung Quaternium-87 als Varisoft W 575 PG von der Firma Evonik bekannt. In der Formel (I) ist als Gegenion Methosulfat dargestellt. Erfindungsgemäß umfasst sind jedoch als Gegenionen auch die Halogenide wie Chlorid, Fluorid, Bromid, oder auch Phosphate.

Besonders vorteilhaft ist, wenn diese Hydrodispersion eine Fettphase aufweist, die zu 95-98% aus kationischen Imidazolinen der Formel (I), insbesondere solchen, deren Fettkette aus Palmöl stammt, mindestens einem Monoalkyltrimethylammonium Salz, mindestens einem langkettigen Fettalkohol sowie auch den Zusatz von einem ersten Silikonöl. Weiterhin ist es bevorzugt, wenn das Verhältnis des Imidazolinquats zu dem Monoalkyltrimethylammoniumquat 2:1 bis 1:2, besonders bevorzugt 1:1 beträgt, wobei das Imidazolinquat in einem Gewichtsverhältnis von mindestens 0,01 Gew. %, bevorzugt 0,5 Gew.%, besonders bevorzugt 0,5 - 2,5 Gew.% vorhanden ist. Besonders vorteilhaft ist die Zugabe von weiteren Silikonölen mit quaternierten Ammonium-Gruppen oder Aminogruppen als getrennte Phase zu der oben gennanten emulgierten Fett- und Wasserphase.

Als langkettige Fettalkohole dienen C₁₄, C₁₆, C₁₈, C₂₂Alkohole bekannt als Myristyl-, Cetyl-, Stearyl- und Behenyl Alkohole, beziehungsweise Kombinationen aus den vorhergenannten Fettalkoholen wie beispielsweise Cetearyl Alkohol. Die Fettalkohole sind in den erfindungsgemäßen Zusammensetzungen in Mengen von 0,5 bis 3,0 Gew.-%, vorzugsweise 0,5 bis 2,0 Gew.-%, besonders bevorzugt 0,5 bis 1,5 Gew.-% bezogen auf die gesamte Zusammensetzung enthalten.

Monoalkyltrimethylammoniumsalze sind Verbindungen mit einer Kettenlänge des Alkylrestes von 16 bis 24 Kohlenstoffatomen. Diese Verbindungen weisen die in der Formel (II) aus CTFA dargestellte Struktur auf, wobei R, R¹, R", und R^{III} für jeweils eine Methylgruppe stehen und R^{III} für einen gesättigten, verzweigten oder unverzweigten Alkylrest mit einer Kettenlänge von 16 bis 24 Kohlenstoffatomen und A- für ein Anion ausgewählt aus den physiologisch verträglichen Anionen ist.

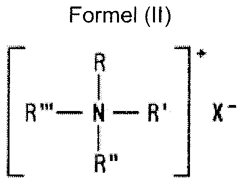

Beispielhaft für das Anion seien die Halogenide, Fluoride, Chloride, Bromide, Sulfate (Methosulfat) der allgemeinen Formel RSO₃⁻worin R die Bedeutung von gesättigtem oder ungesättigtem Alkylresten mit 1 bis 4 Kohlenstoffatomen hat, oder anionische Reste organischer Säuren wie Malest, Fumarat, Oxalat, Tartrat, Citrat, Lactat oder Acetat, genannt.

Beispiele für Verbindungen der Formel (II) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat. Bevorzugt sind Cetyltrimethylammonium- und Behenyltrimethylammoniumsalze. Bevorzugt sind letztere in Form der Methosulfate, Chloride und Bromide. Besonders bevorzugt wird Cetyltrimethylammoniumchlorid und höchst bevorzugt ist Behenyltrimethylammoniumchlorid.

Die Verbindungen der Formel (II) werden in den erfindungsgemäßen Zusammensetzungen in einer Menge von 0,01 bis 5,0 Gew.-% verwendet. Bevorzugt werden 0,1 bis 2,5 Gew.-% verwendet. Besonders bevorzugt sind Mengen von 0,5 bis 1,5 Gew.-%. Die Mengenangaben beziehen sich jeweils auf die gesamte Zusammensetzung.

Die Erfindung kann weiterhin Emulgatoren enthalten. Diese können ausgewählt werden aus der Gruppe Glyceryl Stearate SE, bekannt als Tegin.VS von Evonik, Ceteareth-20 bekannt als Eumulgin B 2 von Cognis, Laureth-4 bekannt als Brij 30 von Croda, Laureth-23 bekannt als Tego Alkanol L 23 P von Evonik, Glyceryl Stearate Citrate bekannt als Imwitor 372 P von Sasol, und Polysorbate 60 bekannt als Tween 60 V von Croda. Auch Silikonemulgatoren können verwendet werden und besonders bevorzugt ist Cyclomethicone mit PEG/PPG-18/18-Dimethicone bekannt als Dow Corning 5225C Formulation Aid. Die bevorzugte Menge der eingesetzten Emulgatoren beträgt 0,01 bis Gew.% 1,0%. Kombinationen von obengenannten

Emulgatoren sind auch geeignet, sowie auch folgende Zusammensetzungen: Glyceryl Isostearate/Isoceteth-20 (Tegin ISO von Evonik/Tego Alkanol IC 20 von Evonik); Ceteareth-20/Glyceryl Stearate + Ceteareth-20 + Cetearyl Alcohol + Cetyl Palmitate + Ceteareth-12 (Eumulgin B 2 von Cognis/Eumulgate SE-PF von Cognis). Eine weitere vorteilhafte Kombination beinhaltet PEG-40 Hydrogenated Castor Oil/Ceteareth-20 (Eumulgin HRE 40 von Cognis/Eumulgin B 2 von Cognis).

Eine weitere Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit der folgenden Strukturformel Formel (III) bekannt als Stearamidopropyl Dimethylamin, Tego Amid S 18 von Evonik Goldschmidt GmbH.

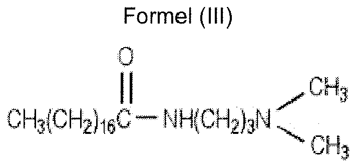

In dieser Erfindung ist es bevorzugt, wenn eine Mischung an Silikonölen verwendet wird, wobei Dimethicone in einer Viskosität von 20cst bis 12500cst, als erste Silikonöl bevorzugt sind, erhalten als Xiameter (R) PMX-200 Silicone Fluid Serie von Dow Corning. Bevorzugte Viskositäten liegen zwischen 50 und 1000cst. Höchst bevorzugt sind Viskositäten um den Bereich von 50 bis 200cst herum. Weitere Handelsnamen für Dimethicone sind unter Wacker Belsil DM Grades, BRB Silicone Oil DM Grades, SF96 Serie (50-1000) sowie auch Baysilone*M (50-1000) Serien von Momentive Performance Materials, DM Fluid Serien (0,65cs bis 3000cs) von Shin-Etsu Chemicals Co bekannt.

Wenn als erste Silikonöl Dimethicone in Form einer Emulsion verwendet wird, dann beträgt die Tröpfchengröße der emulgierten Teilchen erfindungsgemäß 0,01 bis 100 µm, bevorzugt 15 bis 30 µm. Die Teilchengröße wird dabei nach der Methode der Lichtstreuung bestimmt. Diese sind als Dow Corning 5-7139, Dow Corning 2-1352 und Dow Corning 2-1491 Emulsionen bekannt.

Gemäß der Erfindung können anstelle von Dimethicone hoch viskose Dimethiconole in Cyclopentasiloxan, bekannt unter Dow Corning 1501 Fluid, Dow Corning 1411 Fluid, und Silsoft* 1215 von Momentive Performance Materials verwendet werden. Auch hoch viskose Dimethiconole in Dimethicone können verwendet werden, bekannt als Dow Corning 1403 und 1503 Fluide.

Anstelle von Dimethicone als erste Silikon kann Amodimethicone eingesetzt werden. Amodimethicone wird durch folgende Struktur gekennzeichnet: (R=OH oder CH₃, und X: Propyl, Isopropyl, Isobutyl) gemäß Formel (IV).

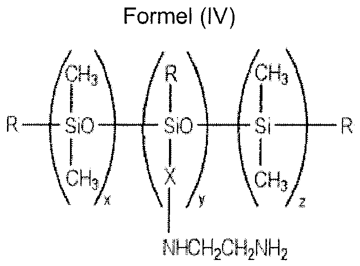

Hierfür bevorzugte Amodimethicone sind als Emulsionen vorhanden und bekannt unter den Handelsnamen:
Dow Corning CE-8170 AF Microemulsion, Dow Corning 929, 939, 949 und 959 Cationic Emulsion, Dow Corning 2-8177 Emulsion, Dow Corning (R) 2-8194 Microemulsion, sowie auch die Wacker Silikone Macroemulsionen Wacker-Belsil ADM 6060 und Wacker-Belsil ADM 6057E, und die Microemulsionen Wacker-Belsil ADM 8020VP und SLM 28040.

Als weiteres Silikonöl dient eine Silikonemulsion. Bevorzugt ist dieses Silikon mit quaternären Ammonium-Gruppen versehen. Hierbei werden Quaternium-16 bekannt als Handelsname Dow Corning 5-7113 und Quaternium-18, ein kationisches Aminosilikon Terpolymer mit quaternären Ammonium-Gruppen und Polyether-Gruppen, als Handelsnamen Silsoft Q und als Mischung mit Cyclopentasiloxan Silsoft Care besonders bevorzugt. Silsoft Q wird auch als Amino Quat Polyether (AB)n Terpolymer genannt. Dabei ist es bevorzugt wenn die Einsatzkonzentration des Aminosilikon Terpolymers zwischen 0,1 und 5 Gew.% beträgt. Besonders vorteilhaft liegt die Einsatzkonzentration bei 0,5 und 2,5 Gew.% Das Verhältnis zwischen dem ersten Silikonöl und dem zweiten Silikon mit quaternären Ammonium Gruppen beträgt vorteilhaft 2.0 :1 bis 1,4 : 1, besonders bevorzugt 1,5:1,0.

Besonders bevorzugte erfindungsgemäße Kombinationen sind in der Spray Formulierung Dimethicone mit Amodimethicone mit Präferenz für Dow Corning 929, 939, 949 und 959 Cationic Emulsionen sowie auch Dimethicone mit einem kationischen Aminosilikon Terpolymer mit quaternären Ammonium-Gruppen und Polyether Gruppenen, beispielsweise Silsoft Q. Eine weitere erfindungsgemäße Kombination wäre Dimethicone mit einem kationischen Aminosilikon Terpolymer mit quaternären Ammonium-Gruppen und Polyether Gruppen und Amodimethicone.

Weiter bevorzugt ist es, UV-Filter besonders bevorzugt Benzophenone-4 zusätzlich hinzuzufügen. Weiter besonders bevorzugt ist es, wenn zusätzlich Oryzanol und optional oder zwingend wenigstens eine Komponente gewählt unter Babassuöl, Jojobaöl, Aloe Vera, Mandelöl, Keratinhydrolysat, dem "Liquid Crystal" Complex ISP Colorflow ^{™} 102, PEG-40 Hydrogenated Castor Oil, Propylenglycol zugesetzt wird.

Erfindungsgemäß sollte die Zusammensetzung bei der Herstellung nicht homogenisiert werden. Alle Komponenten, die sich in der Fettphase befinden, werden etwa zwischen 70 und 90°C, bevorzugt zwischen 80°C und 90°C gebracht. Die Parfümölphase wird zur heißen Fettphase gegeben. Ebenfalls wird die Wasserphase auf die gleiche Temperatur gebracht und beide Phasen unter Rühren ohne Homogenisieren zusammengeführt. Das zweite Silikonöl wird vorzugsweise unterhalb 40°C unter Rühren hinzugefügt.

### FORMELBEISPIELE

Die folgenden Beispiele sollen den Gegenstand der vorliegenden Erfindung erläutern ohne sie zu beschränken.

| **Handelsname** | **Inci** | **Bsp. 1** | **Bsp. 2** | **Bsp. 3** | **Bsp. 4** | **Bsp. 5** | **Bsp. 6** | **Bsp. 7** | **Bsp. 8** |
|---|---|---|---|---|---|---|---|---|---|
| Lanette 18, Cognis, 100% | Stearyl Alcohol | 1,00 | | 0,80 | | 0,90 | 1,00 | 1,00 | |
| Lanette 16, Cognis, 95% | Cetyl Alcohol | | 1,50 | | 2,00 | | | | |
| Lanette-O, Cognis, 100% | Cetearyl Alcohol | | | | | | | | 1,20 |
| Baysilone-Oel M 100, Momen-tive Perfor-mance Mate-rials, 100% | Dimethicone | 1,50 | 2,50 | 1,20 | 2,00 | 1,00 | | 1,00 | 1,7 |
| Genamin KDMP, Clariant, 83% | Behentrimonium Chloride | 1,00 | 0,80 | | 1,50 | 0,90 | 1,00 | 1,00 | 0,50 |
| Dehyquart A-CA, Cognis, 25% | Cetrimonium Chloride | | | 1,20 | | | | | 0,5 |
| Varisoft W 575 PG, Evonik Goldschmidt, 74% | Quaternium-87 | 1,00 | 1,10 | 0,90 | 1,30 | 0,70 | 1,00 | 1,00 | 1,10 |
| Silsoft Q, Mo-mentive Per-formance Mate-rials, 20% | Silicone Quaternium-18+ Trideceth-6+ Trideceth-12 | | 1,50 | | 2,00 | 1,00 | 1,00 | 0,50 | 1,50 |
| Dow Corning 959 Cationic Emulsion, Dow Corning, 50% | Amodimethicone + Trideceth-12 + Cetrimonium Chlo-ride | 1,00 | | 0,70 | | | 1,50 | 1,50 | |
| Tegin VS | Glyceryl Stearate SE | | 0,20 | | | | | | 0,20 |
| Solbrol M, Lanxess, 99,5% | Methylparaben | 0,20 | 0,20 | 0,20 | 0,20 | 0,20 | 0,20 | 0,20 | 0,20 |
| Phenoxyethanol P5, Omya Peralta, 100% | Phenoxyethanol | 0,60 | 0,60 | 0,60 | 0,60 | 0,60 | 0,60 | 0,60 | 0,60 |
| Cremophor CO 40, BASF, 100% | PEG-40 Hydrogenated Castor Oil | 0,90 | 0,70 | 0,90 | 0,70 | 0,90 | 0,90 | 0,90 | 0,70 |
| | Parfum | 0,90 | 0,70 | 0,90 | 0,70 | 0,90 | 0,90 | 0,90 | 0,70 |
| | Aqua | ad 100 | ad 100 | ad 100 | ad 100 | ad 100 | ad 100 | ad 100 | ad 100 |

## Patentansprüche

1. Haarnachbehandlungsmittel in Form einer sprühbaren Hydrodispersion mit Viskosität kleiner als 1000mPas [gemessen mit dem Rheomat R 123 proRheo, Messkörper 1 (Art.Nr. 200191)] enthaltend kationische Imidazoline der Formel 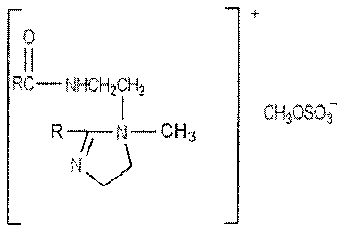 bevorzugt diejenigen, bei denen R ein aliphatischer Rest aus dem Triglycerid Palmöl ist, in Kombination mit Monoalkyltrimethylammonium Salzen, langkettigen Fettalkoholen einem erstem Silikonöl und einem weiteren Silikonöl, das getrennt von dem ersten Silikonöl zu der Hydrodispersion hinzugefügt wird.

2. Mittel nach Patentanspruch 1 **dadurch gekennzeichnet, dass** als kationisches Imidazolin Quaternium-87 verwendet wird.

3. Mittel nach einem der vorangehenden Patentansprüche **dadurch gekennzeichnet, dass** das Massenverhältnis der kationischen Imidazoline zu Monoalkyltrimethylammoniumquat zwischen 2:1 oder 1:2 liegt.

4. Mittel nach einem der vorangehenden Patentansprüche **dadurch gekennzeichnet, dass** die kationischen Imidazoline wenigstens in einem Gewichtsanteil von 0,01% vorhanden ist.

5. Mittel nach einem der vorangehenden Patentansprüche **dadurch gekennzeichnet, dass** als langkettige Fettalkohole C₁₄, C₁₆, C₁₈, C₂₂ Alkohole verwendet werden, besonders bevorzugt Stearyl Alkohol.

6. Mittel nach einem der vorangehenden Patentansprüche **dadurch gekennzeichnet, dass** die langkettigen Fettalkohole in Mengen von 0,5 bis 3,0 Gew.-%, vorzugsweise 0,5 bis 2,0 Gew.-%, besonders bevorzugt 0,5 bis 1,5 Gew.-% bezogen auf die gesamte Zusammensetzung enthalten sind.

7. Mittel nach einem der vorangehenden Patentansprüche **dadurch gekennzeichnet, dass** als Monoalkyltrimethylammoniumsalze Cetyltrimethylammonium- und Behenyltrimethylammoniumsalze, bevorzugt in Form der Methosulfate, Chloride und Bromide, besonders bevorzugt Cetyltrimethylammoniumchlorid und höchst bevorzugt Behenyltrimethylammoniumchlorid eingesetzt werden.

8. Mittel nach einem der vorangehenden Patentansprüche **dadurch gekennzeichnet, dass** die Monoalkyltrimethylammoniumsalze in einer Menge von 0,01 bis 5,0 Gew.-% verwendet werden.

9. Kosmetisches Haarbehandlungsverfahren **gekennzeichnet durch** Aufsprühen eines Mittels nach einem der vorangehenden Patentansprüche und Verzicht auf das Ausspülen während der auf die Anwendung folgenden 24 Stunden.
